# FASCICULE DE BREVET EUROPEEN

(11) **EP 2 227 704 B1**
(45) Date de publication et mention de la délivrance du brevet: **20.04.2011**
(21) Numéro de dépôt: 08856843.1
(22) Date de dépôt: 04.12.2008
(51) Int. Cl.: G01T 1/161, G21G 4/08

(54) **SOURCE RADIOACTIVE FLEXIBLE ETALONNEE**
KALIBRIERTE FLEXIBLE RADIOAKTVE QUELLE
CALIBRATED FLEXIBLE RADIOACTIVE SOURCE

(30) Priorité: 07.12.2007 FR 0759646
(43) Date de publication de la demande: 15.09.2010
(73) Titulaire: Institut de Radioprotection et de Sûreté Nucléaire, 92260 Fontenay aux Roses (FR)
(72) Inventeur: MAULARD, Alain, F-77420 Champs sur Marne (FR)
(74) Mandataire: Ilgart, Jean-Christophe
(86) Numéro de dépôt international: PCT/EP2008/066773
(87) Numéro de publication internationale: WO 2009/071619

(56) Documents cités:
- FR-A- 2 589 058
- US-B1- 6 770 019
- SCHLÄGER, DEDERICHS, LENNARTZ, HILL, HILLE, BABENKO, NESTERENKO, NESTERENKO: "Intercalibration and intervalidation of in-vivo monitors used for whole-body measurements within the framework of a German-Belarussian project" IRPA 11, [Online] 23 mai 2004 (2004-05-23), - 28 mai 2004 (2004-05-28) page 3, XP002470666 Madrid, Espagne Extrait de l'Internet: URL:http://irpa11.irpa.net/pdfs/3a44.pdf> [extrait le 2008-02-27]
- CARLAN, ROCH, BLANCHARDON, FRANCK: "NEW METHOD OF VOXEL PHANTOM CREATION : APPLICATION FOR WHOLE BODY COUNTING CALIBRATION AND PERSPECTIVES IN INDIVIDUAL INTERNAL DOSE ASSESSMENT" INTERNATIONAL CONFERENCE ON RADIATION SHIELDING (ICRS-10) AND THIRTEENTH TOPICAL MEETING ON RADIATION PROTECTION AND SHIELDING PRESENTATION, [Online] 12 mai 2004 (2004-05-12), page 6, XP002470667 Medeira, Protugal Extrait de l'Internet: URL:http://www.itn.pt/ICRS-RPS/oralpdf/Wed nesday12/Session15_2/carlan05.pdf> [extrait le 2008-02-27]

## Description

### DOMAINE TECHNIQUE

L'invention concerne une source radioactive étalonnée qui est flexible et qui peut par exemple être utilisée dans un fantôme équivalent tissus pour réaliser des mesures d'anthropogammamétrie.

### ÉTAT DE LA TECHNIQUE ANTÉRIEURE

L'anthropogammamétrie est la mesure directe in vivo de la radioactivité présente dans l'organisme d'un individu, par exemple à la suite d'une contamination radioactive.

La méthode de mesure généralement utilisée pour mesurer la radioactivité présente dans l'organisme d'un individu consiste à quantifier les rayonnements x et y émis par des radionucléides éventuellement présents dans cet organisme à l'aide d'un détecteur. Cependant, afin de pouvoir déterminer cette quantité de radionucléides, il est nécessaire d'étalonner au préalable le détecteur en fonction du radionucléide considéré. Pour cela, une source étalonnée est placée dans un fantôme physique anthropomorphique. Un fantôme physique ou équivalent tissus est un substitut reproduisant les caractéristiques de l'individu à étudier. Il est réalisé à partir de matériaux présentant des densités et des numéros atomiques proches de ceux des tissus humains, de sorte que le fantôme atténue les rayonnements de la même manière que l'organisme de l'individu à considérer.

Un des fantômes les plus utilisés est le fantôme UP-02T (« IGOR ») qui consiste en un jeu de briques de polyéthylène rectangulaires de masses de 0,88 kg et 0,4 kg qui permettent de simuler différents types de personnes, allant d'un enfant de 10 kg à un adulte de 110 kg. Chaque brique comporte deux trous dans lesquels sont placées des sources radioactives solides et cylindriques de 6 mm de diamètre et de 163 mm de longueur servant à réaliser les étalonnages.

Les sources radioactives étalonnées actuellement utilisées pour le fantôme IGOR sont constituées d'une poudre marquée par un produit radioactif et contenue dans un tube cylindrique en plastique dur.

Le problème est que ces sources, adaptées pour le fantôme IGOR, ne sont plus fabriquées ni commercialisées et qu'aucune source équivalente n'est actuellement proposée par les fournisseurs français et étrangers de sources étalonnées. Les sources étalonnées qui arrivent en fin de vie ne peuvent donc pas être remplacées.

Par ailleurs, ces sources radioactives étalonnées présentent l'inconvénient d'être facilement cassables, ce qui constitue un risque non négligeable de dispersion de la matière radioactive.

De plus, les sources étalonnées glissent librement dans les trous des briques du fantôme, ce qui constitue un risque de chute ou de cisaillement d'une ou de plusieurs sources étalonnées lors de la manipulation du fantôme, entraînant là encore une dispersion de la matière radioactive.

Les inventeurs ont donc cherché à réaliser une source étalonnée de remplacement qui soit incassable et facilement insérable dans un trou, par exemple un trou d'une brique de fantôme, mais sans possibilité de glissement intempestif une fois en place.

### EXPOSÉ DE L'INVENTION

Ce but est atteint grâce à une source radioactive étalonnée, comprenant un contenant et un matériau marqué par au moins un radionucléide, ledit matériau marqué étant contenu dans le contenant et ledit contenant étant constitué en un matériau transparent aux rayonnements émis par ledit au moins un radionucléide, la source étant caractérisée en ce que le matériau marqué est un polymère autodurcissable chimiquement inerte vis-à-vis du matériau constituant le contenant et le contenant est une gaine flexible.

L'invention consiste ainsi à associer une gaine flexible réalisée en un matériau polymère (par exemple une gaine en silicone) à un polymère autodurcissable, inerte vis-à-vis du matériau constituant la gaine flexible (par exemple une résine époxide) et marqué par un ou plusieurs produit(s) radioactif(s) (radionucléides).

Dans le texte, on entend par « polymère autodurcissable » tout polymère qui peut durcir sans apport de chaleur.

Par ailleurs, on entend par « gaine » une enveloppe de forme longiligne et tubulaire. Avantageusement, la gaine a une section essentiellement circulaire.

En utilisant une gaine flexible et un polymère autodurcissable marqué pour remplir la gaine, on obtient une source radioactive étalonnée qui conserve les avantages de la gaine, à savoir sa flexibilité, sa résistance aux chocs et sa capacité à retrouver sa forme initiale après une déformation. Ainsi, étant donné que les risques de rupture de la gaine sont faibles, le risque de dispersion de la matière radioactive incluse dans le polymère autodurcissable est, elle aussi, très faible. Les risques de dispersion de la matière radioactive dans l'environnement lors du transport et de la manipulation de la source étalonnée selon l'invention sont donc grandement diminués.

Par ailleurs, comme la source est flexible, elle peut être utilisée dans des géométries irrégulières.

En outre, comme la gaine flexible est réalisée en un matériau polymère, elle présente l'avantage de pouvoir être conçue avec une forme spécifique qui lui permette, lorsqu'elle est déformée, d'être insérée facilement dans un trou ou un orifice, puis d'y rester bloquée en reprenant sa forme initiale.

Le contenant, qui était un tube en plastique dur dans l'art antérieur, est remplacé par une gaine flexible dans l'invention. Avantageusement, la gaine flexible est un cylindre creux de diamètre essentiellement constant, par exemple un tuyau.

Avantageusement, la gaine flexible est en un matériau polymère, par exemple en élastomère de silicone.

Avantageusement, le polymère autodurcissable du matériau marqué est une résine époxide, par exemple le bisphénol.

Avantageusement, le polymère autodurcissable du matériau marqué comprend 53% de résine époxide, 32% de durcisseur et 15% de liquéfiant, à + ou - 1%. Avantageusement, la résine époxide est l'ARALDITE MY 757®, le durcisseur est l'ARADUR 850 CH® et le liquéfiant est un éther monoéthylique de l'éthylène glycol.

Avantageusement, le matériau marqué comprend du Cobalt 57.

La source radioactive étalonnée selon l'invention peut être utilisée dans de nombreux dispositifs de mesure de la radioactivité. Par exemple, la source peut être insérée dans un fantôme équivalent tissus pour réaliser des mesures d'anthropogammamétrie. Ainsi, l'invention a également pour objet un ensemble comprenant une brique de fantôme équivalent tissus et au moins une source étalonnée telle que décrite précédemment. La brique est un bloc en polymère ayant une face comprenant au moins un trou destiné à recevoir ladite source étalonnée. La source est configurée de manière à avoir au moins une courbure dans le sens de sa longueur, la distance entre la partie la plus haute et la partie la plus basse de la source prise dans le sens de sa largeur étant égale au diamètre intérieur le plus grand dudit trou, de sorte que la source soit en contact avec la paroi du trou en au moins deux points de contact et exerce des forces de pression dirigées de la source vers la paroi au niveau desdits au moins deux points de contact. Par exemple, pour une source présentant une seule courbure s'étendant le long de sa longueur d'une extrémité de la source à l'autre extrémité, la distance entre la partie la plus haute et la partie la plus basse de la source prise dans le sens de la largeur est la distance entre l'apex de la courbure (l'épaisseur de la source étant incluse) et la base de la courbure constituée par une ligne droite rejoignant les deux extrémités de la source.

En fait, comme la source est flexible, elle est configurée de sorte que, dans sa forme initiale (état non déformé), elle présente au moins une courbure, la distance entre la partie la plus haute et la partie la plus basse de la source prise dans le sens de sa largeur étant supérieure au diamètre du trou et que, dans sa forme déformée, la distance entre la partie la plus haute et la partie la plus basse de la source déformée prise dans la sens de sa largeur soit inférieure au diamètre du trou afin que la source puisse être insérée dans le trou. En reprenant sa forme initiale, la source va venir exercer des forces de pression au niveau des points de contact de la source avec la paroi interne du trou. L'avantage de la source selon l'invention est que, comme la source conserve une certaine flexibilité du fait de l'utilisation d'une gaine flexible, il est possible de déformer la source pour l'insérer dans le trou de la brique du fantôme. Une fois insérée dans le trou, la source tend à reprendre sa forme initiale et est maintenue en place en exerçant des forces de pression en certains endroits des parois internes du trou. Ainsi, une fois en place, la source étalonnée ne glisse pas : elle est autobloquante. Avantageusement, le trou de la brique est un trou cylindrique et rectiligne de diamètre essentiellement constant.

Enfin, un objet de l'invention est un procédé de mise en place d'une telle source étalonnée dans un trou d'un bloc. Le procédé de mise en place d'une source radioactive étalonnée dans un trou présent dans une face d'un bloc, comprend les étapes suivantes :
- la fourniture d'une source étalonnée selon l'une quelconque des revendications 1 à 6, ladite source présentant au moins une courbure sur sa longueur,
- la déformation de la source étalonnée jusqu'à ce que la distance entre la partie la plus haute et la partie la plus basse de la source prise dans le sens de sa largeur soit inférieure au plus petit diamètre du trou du bloc,
- l'insertion de la source dans ledit trou et
- le blocage de la source dans ledit trou par retour de la source vers son état non déformé, la distance entre la partie la plus haute et la partie la plus basse de la source prise dans le sens de sa largeur dans son état non déformé étant supérieure au plus petit diamètre du trou.

Une fois placée dans le trou, la source tend à retrouver sa forme initiale et est maintenue en place dans le trou en exerçant une force de pression en certains endroits sur les parois internes du trou.

Avantageusement, la longueur de la source est inférieure ou égale à la longueur du trou du bloc dans lequel elle est insérée. La source étalonnée a ainsi des dimensions adaptées aux dimensions du trou.

Avantageusement, le bloc est une brique de fantôme équivalent tissus en polymère.

### BRÈVE DESCRIPTION DES DESSINS

L'invention sera mieux comprise et d'autres avantages et particularités apparaîtront à la lecture de la description qui va suivre, donnée à titre d'exemple non limitatif, accompagnée des dessins annexés parmi lesquels :
- la figure 1 représente une source étalonnée conforme à l'invention,
- la figure 2 représente une brique d'un fantôme dans laquelle deux sources étalonnées selon l'invention sont en cours d'introduction,
- la figure 3 représente un exemple d'une source étalonnée selon l'invention insérée dans le trou d'une brique de fantôme.

### EXPOSÉ DÉTAILLÉ DE MODES DE RÉALISATION PARTICULIERS

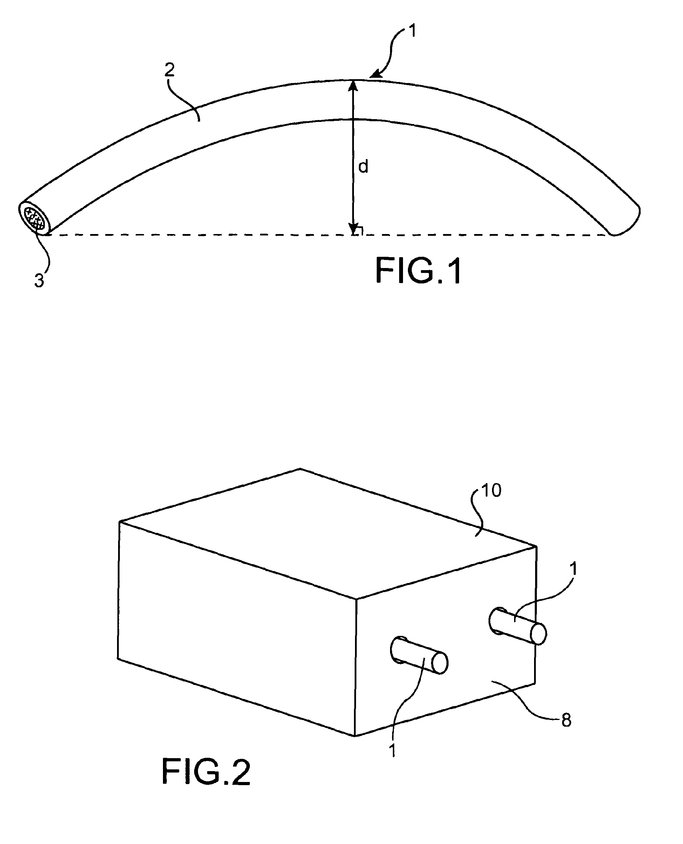

La source étalonnée 1 selon l'invention comporte une gaine flexible 2 dans lequel est injectée une résine marquée par un ou plusieurs radionucléides 3 (voir la figure 1). Les extrémités de la gaine sont recouvertes d'une couche de vernis (non représentée) qui permet d'étanchéifier la source étalonnée.

Cette source étalonnée 1 peut être par exemple insérée dans une brique 10 d'un fantôme équivalent tissus. Dans la figure 2, on a représenté une telle brique comportant, dans l'une de ses faces 8, deux orifices dans lesquels deux sources étalonnées sont en cours d'introduction. Dans cette figure 2, on peut voir que le diamètre de la source est inférieur au diamètre du trou.

La source étalonnée selon l'invention peut être obtenue en suivant les étapes suivantes :
- préparer une solution étalonnée comprenant au moins un radionucléide et préparer un polymère autodurcissable,
- fournir une gaine flexible en matériau transparent aux rayonnements émis par la solution étalonnée et résistant aux chocs,
- mélanger la solution étalonnée et le polymère autodurcissable jusqu'à obtenir une composition homogène,
- remplir la gaine flexible avec la composition homogène,
- laisser reposer la gaine remplie jusqu'à obtenir le durcissement complet de la composition homogène,
- obturer les extrémités de la gaine flexible.

Selon un mode de réalisation, le procédé de fabrication peut comprendre en outre, après l'étape de repos de la gaine remplie et avant l'étape d'obturation, une étape de découpe de la gaine flexible en plusieurs sections constituant chacune une source. Cette découpe de la gaine remplie en plusieurs sections permet au final d'obtenir, en une seule fois, plusieurs sources étalonnées. Avantageusement, les sections sont essentiellement de même longueur.

L'obturation des extrémités de la gaine peut être obtenue par l'application d'un vernis sur lesdites extrémités. Cela permet d'étanchéifier la source étalonnée. On précise que dans le cas où la gaine est découpée en plusieurs sections, la gaine présente alors 2n extrémités, avec n égal au nombre de sections et ce sont ces 2n extrémités qui sont obturées, par exemple par l'application d'un vernis.

De préférence, la solution étalonnée a une masse inférieure ou égale à 2% de la masse de la composition homogène.

Selon un mode de réalisation, la préparation du polymère autodurcissable est réalisée en mélangeant de la résine époxide, un durcisseur et un liquéfiant. Avantageusement, le polymère autodurcissable comprend 53% de résine époxide, 32% de durcisseur et 15% de liquéfiant, à + ou - 1%. Avantageusement, la résine époxide est l'ARALDITE MY 757®, le durcisseur est l'ARADUR 850 CH® et le liquéfiant est un éther monoéthylique de l'éthylène glycol.

Avantageusement, le matériau de la gaine flexible est un polymère, par exemple un élastomère de silicone.

A titre d'illustration, nous allons décrire la fabrication de dix sources étalonnées marquées au Cobalt 57 et adaptées pour des essais de mesure d'anthropogammamétrie dans le fantôme équivalent tissus IGOR.

Comme nous l'avons précisé précédemment, la source étalonnée selon l'invention comprend un polymère autodurcissable marqué à l'aide d'au moins un radionucléide et contenu à l'intérieur d'une gaine flexible.

Le polymère autodurcissable est obtenu en mélangeant trois produits, à savoir une résine chimiquement inerte vis-à-vis du matériau constituant la gaine, par exemple une résine époxide bisphénol A, un durcisseur et un liquéfiant. Le liquéfiant permet d'assurer l'homogénéisation du mélange constitué par la résine, le durcisseur et la solution étalonnée.

Dans le présent exemple, on utilise une résine époxide commercialisée sous la marque ARALDITE MY 757, un durcisseur commercialisé sous la marque ARADUR 850 CH, ces deux produits étant commercialisés par l'entreprise HUNTSMAN ADVANCED MATERIALS (EUROPE) BVBA, et le liquéfiant est par exemple un éther monoéthylique de l'éthylène glycol, chimiquement pur, dont la formule brute est C₂H₅OCH₂CH₂OH.

Ces produits sont versés successivement dans un récipient en plastique et leurs masses sont préalablement déterminées par pesée en respectant les proportions suivantes, exprimées en pourcentage de la masse totale du mélange :
- 53% de résine époxide,
- 32% de durcisseur,
- 15% de liquéfiant,
   ce qui correspond aux quantités suivantes de 15,9 g de résine époxide MY 757, 9,6 g de durcisseur 850 CH et 4,5 g de liquéfiant C₂H₅OCH₂CH₂OH.

Dans ce mélange est ajoutée 0,19281 g d'une solution étalonnée de Cobalt 57, soit 10 420 Bq le 03 décembre 2004, obtenue en mélangeant 50 microgrammes de chlorure de cobalt marqué dans 5 mL d'acide chlorhydrique 0,1 M.

Les différents composants sont ensuite mélangés jusqu'à obtenir une composition homogène. Par exemple, on peut mélanger manuellement les composants en utilisant une spatule en bois.

On précise que la masse de la solution étalonnée est quantifiée par pesée différentielle et doit être inférieure à 2% de la masse totale de la composition homogène, c'est-à-dire du produit comprenant à la fois la solution étalonnée, l'élastomère, le durcisseur et le liquéfiant. Si la solution étalonnée est supérieure à 2% de la masse totale de la composition homogène, la polymérisation n'est pas homogène, ce qui nuit à la qualité de la source.

La composition homogène est ensuite introduite dans une gaine flexible en matériau transparent aux rayonnements émis par le ou les radionucléides présents dans le polymère autodurcissable. La gaine est une gaine flexible réalisée en polymère chimiquement inerte vis-à-vis de son contenant, c'est-à-dire du polymère autodurcissable marqué. Dans notre exemple, la gaine en silicone utilisée est un tuyau cylindrique de diamètre essentiellement constant.

Le matériau de la gaine est choisi de manière à ce que la gaine présente une bonne tenue à la lumière solaire et à l'ozone, et de manière générale, à tous les facteurs habituels de vieillissement des polymères.

Le matériau de la gaine est également choisi de manière à ce que la gaine conserve ses propriétés physiques, mécaniques et électriques dans la gamme de température dans laquelle la source sera utilisée ; par exemple, le matériau de la gaine conserve lesdites propriétés entre -20 et + 200°C en continu.

La gaine est par exemple réalisée en élastomère de silicone.

Afin d'éviter des inclusions de bulles d'air dans le produit final, la composition homogène est laissée à reposer pendant un certain temps, de préférence 1 heure, avant d'effectuer le remplissage de la gaine flexible. Le temps de repos est choisi de manière à ce que le polymère autodurcissable marqué ait suffisamment dégazé, mais qu'il n'ait pas trop durci : un temps d'attente de 1 heure est un bon compromis avec le type de polymère, de durcisseur et de liquéfiant utilisés dans cet exemple.

Puis, après avoir reposé pendant une heure, la composition homogène est injectée dans une gaine souple du type tuyau en élastomère de silicone mesurant deux mètres de long, 5,5 mm de diamètre extérieur et 3 mm de diamètre intérieur, en créant une dépression dans la gaine à l'aide d'une pompe péristaltique (la vitesse de pompage est réglée à 7 mL par min). L'injection par pompage est arrêtée lorsque la composition homogène arrive à environ 5 cm de la valve de la pompe péristaltique. On place alors la gaine remplie sur un support plan, si besoin en la fixant sur le support, et on relève en position verticale les deux extrémités de la gaine afin d'empêcher la composition homogène de s'écouler hors de la gaine.

On laisse la gaine remplie reposer afin que la composition homogène, et en particulier le polymère autodurcissable qu'elle contient, puisse dégazer et se durcir.

Au bout de 72 heures, le durcissement de la composition homogène est complet : l'aspect de la composition homogène dans la gaine est satisfaisant (sans bulle et de couleur homogène).

La gaine est alors découpée en dix sections mesurant chacune 163 mm de long. Les dix sections forment ainsi dix sources étalonnées ayant chacune une longueur de 163 mm et un diamètre extérieur de 5,5 mm.

Puis, on recouvre les extrémités de chacunes des sections avec une couche de vernis afin de garantir l'étanchéité de chaque source cylindrique étalonnée.

Etant donné que la composition homogène, une fois durcie, reste cependant flexible puisqu'elle est placée au sein d'une gaine flexible, la source (gaine remplie) conserve elle aussi un caractère flexible. Ainsi, même lorsque la gaine est endommagée (ce qui est beaucoup plus rare qu'avec un tube en plastique dur), le contenu de la gaine (c'est-à-dire la composition homogène marquée) ne s'écoule et ne se répand pas hors de la gaine, contrairement aux poudres de l'art antérieur, qui se dispersent facilement. Au final, on obtient donc des sources radioactives étalonnées souples et étanches.

Ces dix sources sont ensuite pesées et leur radioactivité est mesurée. Pour cela, les sources sont tour à tour positionnées dans le fond d'une boîte en plastique adaptée pour une mesure en spectrométrie gamma. La boîte est placée à 5 cm d'un détecteur gamma, qui reçoit et comptabilise les photons gamma provenant du cobalt 57 des sources (raie principale à 122 keV), pendant un temps de mesure de 4 200 secondes.

On détermine ensuite l'activité de chaque source. Pour cela, on soustrait à la masse d'une source déterminée la masse moyenne d'une gaine vide de même longueur pour obtenir la masse de composition homogène comprise dans la gaine. L'activité est obtenue en multipliant l'activité totale incorporée dans la totalité de la composition homogène par la masse de composition homogène présente dans la gaine en question, et en divisant par la masse totale de composition homogène préparée.

Les résultats sont présentés dans le tableau 1 ci-dessous. On précise qu'on a pris comme masse moyenne de la gaine vide une valeur égale à 3,0028 g.

A la lecture de ces résultats, on constate que les sources étalonnées selon l'invention sont sensiblement identiques. Le procédé de fabrication est donc validé.

**Tableau 1**

| N° de la gaine | Masse totale de la source étalonnée (g) | Masse de résine (g) | Nombre de coups totalisés en 4200 secondes | Incertitude de comptage en coups | Activité mesurée le 20/03/2006 (Bq) | Nombre de coups par gramme de résine |
|---|---|---|---|---|---|---|
| 1 | 4,4770 | 1,4742 | 10361 | 221,6 | 145,4 | 7028 |
| 2 | 4,4176 | 1,4148 | 9965,2 | 222,9 | 139,9 | 7044 |
| 3 | 4,4643 | 1,4615 | 10403 | 223,7 | 146,0 | 7118 |
| 4 | 4,4795 | 1,4767 | 10388 | 223,3 | 145,8 | 7034 |
| 5 | 4,5534 | 1,5506 | 10445 | 228,4 | 146,6 | 6736 |
| 6 | 4,5408 | 1,5380 | 10663 | 222,9 | 149,3 | 6913 |
| 7 | 4,5501 | 1,5473 | 10606 | 222,9 | 148,8 | 6 855 |
| 8 | 4,5317 | 1,5289 | 10271 | 218,6 | 144,1 | 6718 |
| 9 | 4,5099 | 1,5071 | 10321 | 221,1 | 144,9 | 6848 |
| 10 | 4,5188 | 1,5160 | 10355 | 217,5 | 145,3 | 6830 |
| **Moyenne** | | **1,5015** | **10375** | | **145,6** | **6736** |
| **Ecart-type** | | **0,0478** | **175,5** | | **2,459** | |

Un des avantages des sources étalonnées selon l'invention est qu'elles permettent une meilleure sécurité d'utilisation, ne serait ce que lors de leur manipulation, par exemple lors de l'insertion des sources étalonnées dans les briques d'un fantôme pour réaliser l'étalonnage des installations de mesure anthropogammamétrique. En effet, comme les sources étalonnées sont beaucoup plus résistantes aux chocs que les sources connues, la radioprotection est améliorée lors de phases de manipulation et de transport des sources. Les sources peuvent facilement être manipulées et déplacées sur les différentes installations de mesures anthropogammamétriques situées sur le territoire français, mais également à l'étranger, et on peut ainsi réaliser des tests interlaboratoires pour comparer des résultats obtenus en divers endroits géographiques.

Un autre avantage est que la source étalonnée obtenue répond aux besoins exprimés par son futur utilisateur. En effet, dans l'exemple illustré ci-dessus, la solution étalonnée introduite dans le polymère autodurcissable est réalisée à partir de Cobalt 57. Mais le polymère autodurcissable peut tout à fait contenir un autre élément radioactif ou plusieurs radionucléides différents, tel que le césium 137, le baryum 133, le cobalt 60...

De même, on peut choisir la quantité de radionucléide(s) que l'on souhaite introduire dans le polymère autodurcissable, ainsi que l'intensité de son activité.

Par ailleurs, en choisissant le diamètre extérieur de la gaine flexible, ainsi que la longueur des sections de gaine, on peut obtenir des sources cylindriques étalonnées ayant des diamètres et des longueurs adaptées à l'usage envisagé.

Les sources étalonnées selon l'invention peuvent ainsi, par exemple, être placées dans n'importe quels fantômes IGOR utilisés dans les installations de mesures anthropogammamétriques existantes.

On peut également prévoir de fabriquer des sources étalonnées destinées à être introduites dans des fantômes ayant des ouvertures plus étroites ou plus larges que celles des fantômes IGOR.

Les sources selon l'invention peuvent par exemple être utilisées pour réaliser des intercomparaisons entre laboratoires.

D'autre part, comme les sources selon l'invention peuvent être fabriquées avec des diamètres et des longueurs différents, il est possible d'utiliser ces sources pour réaliser l'étalonnage d'installation de mesure utilisant d'autres types de fantôme que le fantôme IGOR, ces fantômes ayant des trous de diamètres plus grands ou plus petits que ceux des fantômes IGOR.

La source peut posséder une forme initiale (état non déformé) présentant une ou plusieurs courbures. Par exemple, la source peut avoir une forme de cylindre présentant deux courbures dans un plan passant par l'axe du cylindre. En modifiant la ou les courbures de la source, on peut alors l'insérer dans le trou d'un fantôme. Par exemple, si le trou du fantôme dans lequel on souhaite insérer la source est un cylindre rectiligne de diamètre constant, la ou les courbures de la source peuvent être atténuées de sorte que la source puisse être insérée dans le trou. Une fois insérée, la source va tendre à reprendre sa forme initiale et va ainsi rester bloquée dans le trou.

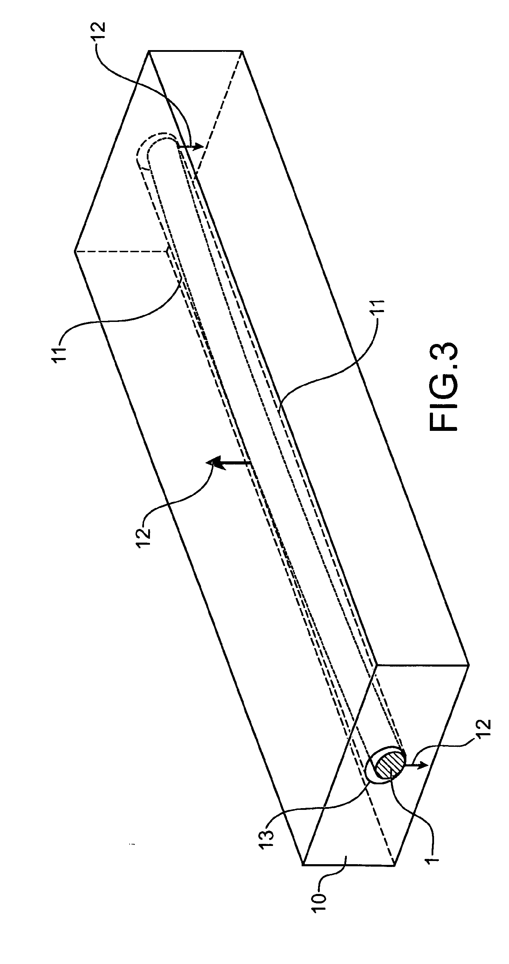

Selon un exemple d'application représenté dans la figure 1, la source est un cylindre comportant une seule courbure s'étendant sur toute la longueur de la source, c'est-à-dire d'une extrémité à l'autre de la source, la courbure ayant une flèche supérieure au diamètre du trou dans laquelle on souhaite introduire la source. La flèche est ici la distance entre l'apex de la courbure, c'est-à-dire la partie la plus haute de la source prise dans le sens de sa largeur (l'épaisseur de la source étant incluse dans cette distance), et la base de la source (ligne droite passant par les deux extrémités de la source), la distance entre l'apex et la base étant bien entendu prise le long d'une ligne droite perpendiculaire à la base (distance d dans la figure 1). La source est déformée de sorte que la flèche de la courbure soit inférieure au diamètre du trou, puis la source est insérée dans ledit trou. Une fois insérée, la source tend à reprendre sa forme initiale dans l'orifice 13 de la brique. Etant donné que la flèche de la courbure de la source dans sa forme initiale est supérieure au diamètre du trou, certaines parties de la source vont venir en contact avec les parois 11 de l'orifice 13 en y exerçant des forces de pression. En l'occurrence, comme la source présente une seule courbure s'étendant sur l'ensemble de la longueur de la source, il y a au final trois points de contact au niveau desquels s'exercent trois forces de pression 12 sur la paroi interne 11 de l'orifice, à savoir deux points de contact situés aux extrémités de la surface latérale de la source et au niveau desquels s'exercent deux forces de pression dirigées de la source vers la paroi interne, et un point de contact central au niveau duquel s'exerce une force de pression dirigée de la source vers la paroi interne, cette dernière force de pression étant de sens opposé au sens des deux forces de pression s'exerçant aux extrémités de la source (voir la figure 3). La source est ainsi bloquée dans le trou : elle est auto-bloquante.

En variant la longueur et le diamètre de la source lors de sa fabrication et/ou en variant sa forme initiale (une ou plusieurs courbures plus ou moins prononcées), en prenant garde toutefois à ce que la source conserve son caractère flexible, on peut ainsi réaliser des sources étalonnées auto-bloquantes qui peuvent facilement être insérées dans les trous des briques de n'importe quel fantôme et y rester bloquées.

## Revendications

1. Source radioactive étalonnée (1), comprenant un contenant et un matériau marqué (3) par au moins un radionucléide, ledit matériau marqué étant contenu dans le contenant et ledit contenant étant constitué en un matériau transparent aux rayonnements émis par ledit au moins un radionucléide, la source étant **caractérisée en ce que** le matériau marqué est un polymère autodurcissable chimiquement inerte vis-à-vis du matériau constituant le contenant et le contenant est une gaine flexible (2).

2. Source étalonnée selon la revendication 1, dans lequel la gaine flexible (2) est un cylindre creux de diamètre essentiellement constant.

3. Source étalonnée selon la revendication 1, dans lequel la gaine flexible (2) est en un matériau polymère, par exemple en élastomère de silicone.

4. Source étalonnée selon la revendication 1, dans lequel le polymère autodurcissable du matériau marqué (3) est une résine de type époxide. ,

5. Source étalonnée selon la revendication 4, dans lequel le polymère autodurcissable comprend 53% de résine époxide, 32% de durcisseur et 15% de liquéfiant, à + ou - 1%.

6. Source étalonnée selon la revendication 1, dans lequel le matériau marqué (3) comprend du Cobalt 57.

7. Ensemble comprenant une brique de fantôme équivalent tissus (10) et au moins une source étalonnée selon l'une quelconque des revendications 1 à 6, la brique étant un bloc en polymère dont une face (8) comprend un trou (13) destiné à recevoir ladite source étalonnée et la source étant configurée de manière à avoir au moins une courbure dans le sens de sa longueur, la distance entre la partie la plus haute et la partie la plus basse de la source prise dans le sens de sa largeur étant égale au diamètre intérieur le plus grand dudit trou, de sorte que la source soit en contact avec la paroi (11) du trou en au moins deux points de contact et exerce des forces de pression (12) dirigées de la source vers la paroi au niveau desdits au moins deux points de contact.

8. Ensemble selon la revendication 7, dans lequel le trou (13) est un trou cylindrique et rectiligne de diamètre essentiellement constant.

9. Procédé de mise en place d'une source radioactive étalonnée dans un trou présent dans une face d'un bloc, comprenant :
- la fourniture d'une source étalonnée (1) selon l'une quelconque des revendications 1 à 6, ladite source présentant au moins une courbure sur sa longueur,
- la déformation de la source étalonnée (1) jusqu'à ce que la distance entre la partie la plus haute et la partie la plus basse de la source prise dans le sens de sa largeur soit inférieure au plus petit diamètre du trou (13) du bloc,
- l'insertion de la source dans ledit trou et
- le blocage de la source dans ledit trou par retour de la source vers son état non déformé, la distance (d) entre la partie la plus haute et la partie la plus basse de la source prise dans le sens de sa largeur dans son état non déformé étant supérieure au plus petit diamètre du trou.

10. Procédé de mise en place d'une source radioactive étalonnée dans un trou présent dans une face d'un bloc selon la revendication 9, dans lequel le bloc est une brique de fantôme équivalent tissus en polymère.

## Claims

1. Calibrated radioactive source (1), comprising a container and a material labelled (3) by at least one radionuclide, said labelled material being contained in the container and said container being made of a material transparent to the radiation emitted by said at least one radionuclide, the source being **characterised in that** the labelled material is a self-hardening polymer chemically inert relative to the material used for the container and **in that** the container is a flexible sheath (2).

2. Calibrated source according to claim 1, wherein the flexible sheath (2) is a hollow cylinder of essentially constant diameter.

3. Calibrated source according to claim 1, wherein the flexible sheath (2) is made of a polymer material, for example silicone elastomer.

4. Calibrated source according to claim 1, wherein the self-hardening polymer of the labelled material (3) is an epoxy type resin.

5. Calibrated source according to claim 4, wherein the self-hardening polymer comprises 53% of epoxy resin, 32% of hardener and 15% of liquefier, to + or - 1%.

6. Calibrated source according to claim 1, wherein the labelled material (3) comprises cobalt 57.

7. Assembly comprising a brick of a tissue-equivalent phantom (10) and at least one calibrated source according to any of claims 1 to 6, the brick being a polymer block, one face (8) of which comprises a hole (13) intended to receive said calibrated source and the source being configured so as to have at least one curvature in the direction of its length, the distance between the highest part and the lowest part of the source taken in the direction of its width being equal to the largest internal diameter of said hole, so that the source is in contact with the wall (11) of the hole in at least two contact points and exerts pressure forces (12) directed from the source to the wall at the level of said at least two contact points.

8. Assembly according to claim 7, wherein the hole (13) is a cylindrical and rectilinear hole of essentially constant diameter.

9. Method for placing a calibrated radioactive source in a hole present in one face of a block, comprising:
- the provision of a calibrated source (1) according to any of claims 1 to 6, said source having at least one curvature over its length,
- the deformation of the calibrated source (1) until the distance between the highest part and the lowest part of the source taken in the direction of its width is less than the smallest diameter of the hole (13) of the block,
- the insertion of the source into said hole and
- the blockage of the source in said hole by return of the source to its non-deformed state, the distance (d) between the highest part and the lowest part of the source taken in the direction of its width in its non-deformed state being greater than the smallest diameter of the hole.

10. Method for placing a calibrated radioactive source in a hole present in one face of a block according to claim 9, wherein the block is a brick of a tissue-equivalent phantom made of polymer.

## Patentansprüche

1. Kalibrierte radioaktive Quelle (1), einen Behälter und ein durch wenigstens ein Radionuklid markiertes Material (3) umfassend, wobei das markierte Material in dem Behälter enthalten ist und der genannte Behälter aus einem für die Strahlen durchlässigen Material ist, die das genannte wenigstens eine Radionuklid abstrahlt, wobei die Quelle
**dadurch gekennzeichnet ist, dass** das markierte Material ein selbsthärtendes Polymer ist, das chemisch inert ist gegenüber dem den Behälter bildenden Material, und der Behälter dabei eine flexible Hülle (2) ist.

2. Kalibrierte Quelle nach Anspruch 1, bei der die flexible Hülle (2) ein hohler Zylinder von im Wesentlichen konstantem Durchmesser ist.

3. Kalibrierte Quelle nach Anspruch 1, bei der die flexible Hülle (2) aus einem polymeren Material ist, zum Beispiel aus Siliconelastomer.

4. Kalibrierte Quelle nach Anspruch 1, bei der das selbsthärtende Polymer des markierten Materials (3) ein Harz des Epoxidtyps ist.

5. Kalibrierte Quelle nach Anspruch 4, bei der das selbsthärtende Polymer 53% Epoxidharz, 32% Härter und 15% Verflüssigungsmittel enthält, mit + oder- 1 %.

6. Kalibrierte Quelle nach Anspruch 1, bei der das markierte Material (3) Kobalt 57 enthält.

7. System, einen Gewebeäquivalenz-Phantomstein (10) und wenigstens eine kalibrierte Quelle nach einem der Ansprüche 1 bis 6 umfassend, wobei der Phantomstein ein Polymerblock ist, dessen eine Seite (8) ein zur Aufnahme der genannten kalibrierten Quelle bestimmtes Loch (13) enthält und die Quelle dabei so geformt ist, dass sie wenigstens eine Krümmung in der Richtung ihrer Länge bzw. über ihre Länge aufweist, wobei der Abstand zwischen dem höchsten Teil und dem tiefsten Teil der Quelle, gemessen in der Richtung ihrer Breite, gleich dem größten Innendurchmesser des genannten Lochs ist, so dass die Quelle in wenigstens zwei Kontaktpunkten mit der Wand (11) des Lochs Kontakt hat und Druckkräfte (12) ausübt, von der Quelle in Richtung Wand, in Höhe der genannten wenigstens zwei Kontaktpunkte.

8. System nach Anspruch 7, bei dem das Loch (13) ein zylindrisches und geradliniges Loch von im Wesentlichen konstantem Durchmesser ist.

9. Verfahren zur Einsetzung einer kalibrierten radioaktiven Quelle in ein auf einer Seite eines Blocks vorhandenes Loch, umfassend:
- Bereitstellung einer kalibrierten Quelle (1) nach einem der Ansprüche 1 bis 6, wobei die genannte Quelle wenigstens eine Krümmung über ihre Länge aufweist,
- Deformation der kalibrierten Quelle (1), bis der Abstand zwischen dem höchsten Teil und dem tiefsten Teil der Quelle, gemessen in der Richtung ihrer Breite, niedriger ist als der kleinste Durchmesser des Lochs (13) des Blocks,
- Einführung der Quelle in das genannte Loch, und
- Blockierung der Quelle in dem genannten Loch durch Rückkehr der Quelle in ihren undeformierten Zustand, wobei der Abstand (d) zwischen dem höchsten Teil und dem tiefsten Teil der Quelle, gemessen in der Richtung ihrer Breite, in ihrem undeformierten Zustand größer ist als der kleinste Durchmesser des Lochs.

10. Verfahren zur Einsetzung einer kalibrierten radioaktiven Quelle in ein in einer Seite eines Blocks vorhandenes Loch nach Anspruch 9, bei dem der Block ein Gewebeäquivalenz-Phantomstein aus Polymer ist.
